Europäisches Patentamt

European Patent Office

Office européen des brevets

(11) Veröffentlichungsnummer: **0 266 727 B1**

# EUROPÄISCHE PATENTSCHRIFT

(45) Veröffentlichungstag der Patentschrift: 24.07.91

(21) Anmeldenummer: 87116111.3

(22) Anmeldetag: 02.11.87

(51) Int. Cl.⁵: **C07C 9/04**, C07C 1/12, C07C 31/04, C07C 29/15, B01J 23/76, B01J 23/44

Die Akte enthält technische Angaben, die nach dem Eingang der Anmeldung eingereicht wurden und die nicht in dieser Patentschrift enthalten sind.

(54) **Verfahren zur Hydrierung von Kohlendioxid und Katalysator.**

(30) Priorität: 05.11.86 CH 4412/86

(43) Veröffentlichungstag der Anmeldung:
**11.05.88 Patentblatt 88/19**

(45) Bekanntmachung des Hinweises auf die Patenterteilung:
**24.07.91 Patentblatt 91/30**

(84) Benannte Vertragsstaaten:
**CH DE FR GB LI**

(56) Entgegenhaltungen:
**EP-A- 0 173 088**
**US-A- 3 876 557**

**Journal of catalysis, Band 96, 1985, San Diego M. SHIBATA et al. "Methanol synthesis reaction over coppergroup IV metal amorphous alloys as catalyst precursor" Seiten 296-298**

**Chemistry letters, 1983, The Chemical Society of Japan A. YOKOHAMA et al. "An active methanation catalyst prepared from an amorphous Pd35Zr65 alloy" Seiten 195-198**

(73) Patentinhaber: **LONZA AG**

**Gampel/Wallis(CH)**

(72) Erfinder: **Baiker, Alfons, Dr.**
**Rietgrabenstrasse 63**
**CH-8152 Opfikon (Kanton Zurich)(CH)**
Erfinder: **Linden, Gerd, Dr.Dipl.- Chem.**
**An den Linden 4**
**BE-4780 St.Vith(BE)**

(74) Vertreter: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

Chemical abstracts, Band 105, Nr. 18, 3. November 1986, Columbus, Ohio, USA A. BAIKER et al. "Novel hydrogenation catalyst prepared from an amorphous Cu70Zr30 precursor" Seite 421, Zusammenfassung-Nr. 159 431z

Chemical abstracts, Band 94, Nr. 20, 18. Mai 1981, Columbus, Ohio, USA F.L.H.M. SPIT ET AL. "Hydrogen sorption by the nickel-zirconium metallic glass Ni64Zr36 and by related crystalline compounds" Seite 183, Zusammenfassung-Nr. 159 819 m

Patent abstracts of Japan, unexamined applications, Field C, Band 7, Nr. 281, 15. Dezember 1983 THE PATENT OFFICE JAPANESE GOVERNMENT Seite 71 C 200

**Beschreibung**

Die Erfindung betrifft ein Verfahren zur Hydrierung von Kohlendioxid in Gegenwart von Katalysatoren.

Es ist bekannt, Fällungskatalysatoren aus Kupfer und Zinkoxiden zur Hydrierung von Kohlenmonoxid zu Methanol zu verwenden. (R.G. Hermann et al., J.Catal., 56, 407-429, (1979).)

In der US-A-3 876 557 ist außerdem erwähnt, daß sich ein Nickel-Zirkonium-Fällungskatalysator für die Hydrierung von Fettsäuren und pflanzlichen Ölen eignet.

Es ist auch bekannt, Kohlenmonoxid in Gegenwart von amorphen Metallbändern aus Eisen und Nickel mit Phosphor- und Borzusätzen zu $C_1$-$C_3$ Kohlenwasserstoffen umzusetzen. (Yokohama et al, Journal of Catalysis, 68, 355-361, (1981).)

Vom gleichen Verfasser stammt der Vorschlag, eine amorphe Metallegierung der Zusammensetzung $Pd_{35}Zr_{65}$ als Methanisierungskatalysator für CO und $H_2$ einzusetzen. (Yokohama et al, Chemistry letters, The Chemical Society of Japan, 195-198, (1983).)

Weiters wurde über Verwendung von amorphen $Cu_{70}Zr_{30}$-Legierungen für die Hydrierung von CO zu Methanol berichtet. (M. Shibata, Y. Okbayashi, N. Kawaka, T. Masumo und K. Aoki, J.Catal., 96, (1985) 296.)

Gemäß Patent Abstracts of Japan, Band 7 (1988), Seiten 71C bis 200, Nr. 58-159847 eignet sich eine Reihe von amorphen Metalllegierungen für die Hydrierung von Kohlenmonoxid.

Schließlich beschreibt die EP-A-173 088 ein Verfahren zur Herstellung von katalytisch wirksamen, glasig erstarrten Metallen, u.a. solchen der Summenformeln $Cu_{70}Zr_{30}$ und $Ni_{64}Zr_{36}$.

Der Gegenstand der vorliegenden Erfindung ist ein Verfahren zur Hydrierung von Kohlendioxid in Gegenwart von Katalystoren, das dadurch gekennzeichnet ist, daß Kohlendioxid und Wasserstoff mit einem entweder bei 200 bis 350°C und $10^5$ bis $10^6$ Pa in einem Kohlendioxid und Wasserstoff in einem Verhältnis von Kohlendioxid zu Wasserstoff von 1:1 bis 1:4 Volumenteilen enthaltenden Gasstrom oder bei 200 bis 350°C im Wasserstoffstrom aktivierten Katalysator aus der Reihe der zirkonhaltigen Metallegierungen mikrokristalliner bis amorpher Struktur der Formel $Cu_{70}Zr_{30}$, $Ni_{64}Zr_{36}$ oder $Pd_{25}Zr_{75}$ in Berührung gebracht werden.

Eine der Metallegierungen, die vorzugsweise angewendet wird, ist amorphes $Cu_{70}Zr_{30}$. Diese Legierung eignet sich besonders für die Hydrierung von Kohlendioxid. Als Reaktionsprodukt entsteht mit hoher Selektivität Methylalkohol. Weitere bevorzugte Metallegierungen sind das amorphe $Ni_{64}Zr_{36}$ sowie das mikrokristalline bis amorphe $Pd_{25}Zr_{75}$, das ebenfalls Gegenstand der vorliegenden Erfindung ist.

Als Reaktionsprodukt entsteht mit diesen Katalysatoren - mit hoher Selektivität - Methan.

Die erfindungsgemäß eingesetzten Metallegierungen werden erhalten durch Schmelzen der einzelnen Elemente, vorzugsweise unter einem Schutzgas wie Argon, und erneutem Schmelzen der Vorlegierung und anschliessendes Abschrekken mit Kühlraten von $10^3$ K/sec. bis $10^9$ K/sec. und vorzugsweise $10^6$ K/sec. bis $10^9$ K/sec. Geeignete Verfahren sind an sich bekannt und beispielhaft können für die Herstellung von Bändern das Schmelz-Spinn-Verfahren genannt werden. Dazu wird die Vorlegierung unter Schutzgas in einem Tiegel geschmolzen und die Schmelze im Strahl auf eine gekühlte, rotierende Trommel, aus beispielsweise Kupfer oder Eisen, gegossen und die amorphe Metallegierung in Bandform abgezogen. Für die Herstellung von Blättchen kann weiters das "Splat- cooling"-Verfahren erwähnt werden.
Pulvrige Metallegierungen lassen sich beispielsweise durch Mahlen der Bänder oder Blättchen herstellen.

Derartige amorphe Legierungen werden auch als glasig erstarrte Metalle (glassy metals) bezeichnet und sind nicht immer exakt beschreibbar, da die Metalle in ihren hochdispersen Formen in amorphen als auch in kristallinen Bezirken von nur einigen Atomen vorliegen können.

Die amorphen Metalle bilden eine neue Substanzklasse, mit überraschenden und interessanten neuen katalytischen Eigenschaften. Genannt seien hier nur Metastabilität und ungesättigte Koordinationssphäre der Metallatome. Wichtig ist auch die Möglichkeit einer sehr homogenen Mischung verschiedener Atomsorten, und somit die Darstellung von Perkolationssystemen.

Für das erfindungsgemässe Verfahren werden die Metallegierungen in Form von Bändern, Formkörpern mit möglichst grosser Oberfläche pro Volumeneinheit oder als Pulver eingesetzt. Die Aktivierung der Metallegierungen erfolgt entweder in situ im Reaktionsstrom oder durch Aktivierung im Wasserstoffstrom. Die "in situ"-Aktivierung dauert in der Regel etwa 4 bis 50 Stunden, die Aktivierung im Wasserstoffstrom etwa 2 bis 20 Stunden. Die Aktivierungstemperaturen liegen bei 200 bis 350°C.

Als Ausgangsmaterialien werden Kohlendioxid und Wasserstoff im Verhältnis von Kohlendioxid zu Wasserstoff von 1:1 bis 1:4 Volumenteilen eingesetzt.

Gegebenenfalls kann auch Kohlendioxid in Mischung mit Anteilen von Kohlenmonoxid angewendet werden.

Die Reaktion kann bei Normaldruck, gegebenenfalls auch bei Ueber- oder Unterdruck, durchgeführt werden. Ein geeigneter Arbeitsbereich liegt bei-

spielsweise bei $10^5$ bis $10^6$ Pa. Die zweckmässig eingehaltene Reaktionstemperatur kann 150 bis 450 °C, vorzugsweise 200 bis 350 °C betragen. Die Katalysatoren arbeiten wie vorerwähnt mit hoher Selektivität bezüglich Methan und fallweise Methanol und Dimethylether. $Cu_{70}Zr_{30}$ weist tendenziell eine hohe Selektivität bezüglich Methanol auf, während $Pd_{25}Zr_{75}$ und $Ni_{64}Zr_{36}$ tendenziell eine hohe Selektivität bezüglich Methan zeigen. Typische Selektivitäten sind beispielsweise für Methanol und Dimethylether mit $Cu_{70}Zr_{30}$ ca 80 %, für Methan mit $Ni_{64}Zr_{36}$ rund 95 % und mit $Pd_{25}Zr_{75}$ grösser als 90 %.

Beispiele

Katalyseexperimente

Die Kontakte (0,25 bis 1,00 g) wurden in einer Reaktionsdurchflussapparatur, in einem Mikroreaktor aus rostfreiem Stahl, bei einer Raumgeschwindigkeit von 12000 bis 3000 $h^{-1}$ 1, mit Wasserstoff, Synthesegas ($H_2$:CO = 2:1) behandelt. Die Gase wurden nachgereinigt. Die Versuche wurden zumeist bei 1,05 MPa durchgeführt. Eine allfällige $H_2$-Vorbehandlung wurde bei 0,1 MPa durchgeführt. Einem Wechsel des Gases oder einem Abbruch des Versuchs ging ein Sinken der Temperatur auf Raumtemperatur voraus; es schloss sich das Umschalten auf das neue Gas an, Spülen des Reaktors und Hochfahren der Temperatur auf den gewünschten Wert.

Vergleichsbeispiele mit Fällungskatalysatoren

   a) CuO/ZnO (3:7)
   b) CuO/ZnO (7:3)
   c) $CuO/ZrO_2$ (3:7)
   d) $CuO/ZrO_2$ (7:3)

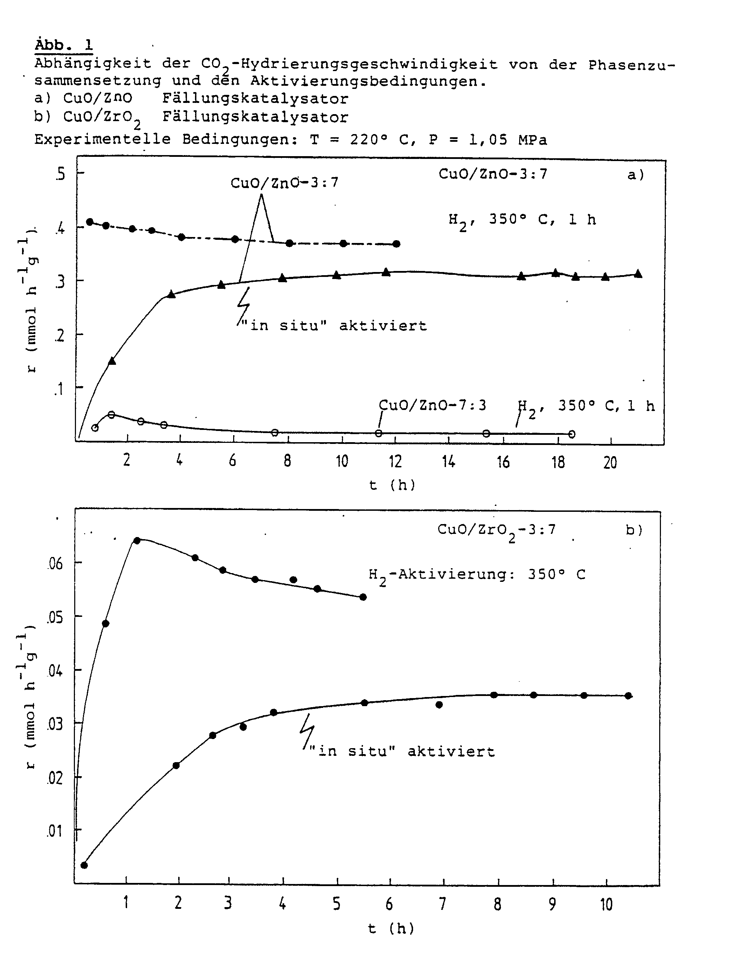

Die Fällungskatalysatoren entstanden nach folgender Vorschrift. Zu 1 Liter wässriger Lösung, enthaltend 0,3 (bzw. 0,7) Mol $Cu(NO_3)_2$ und 0,7 (bzw. 0,3) Mol $Zn(NO_3)_2$ oder $ZrCl_4$, wurde tropfenweise unter Rühren bei 90 °C eine $1m$-$Na_2CO_3$-Lösung zugegeben bis ein pH-Wert von 7 erreicht wurde. Anschliessend wurde während 2 Stunden unter Rühren abkühlen gelassen. Der Niederschlag wurde filtriert und gründlich gewaschen und anschliessend T-programmiert (50 °C/min.) auf 350 °C geheizt und dortselbst 3 Stunden belassen. Es wurde nun gefunden, dass bei der Hydrierung von $CO_2$ bei einer Temperatur von 220 °C und einem Druck von 1 MPa der CuO/ZnO-Katalysator mit der Zusammensetzung 3:7 wesentlich aktiver ist als CuO/ZnO (7:3). Einen ebenso drastischen Unterschied der Aktivitäten fand man bei $CuO/ZrO_2$-Fällungskatalysatoren Während $CuO/ZrO_2$ (3:7) eine merkliche Aktivität zeigte, war

sie für $CuO/ZrO_2$ (7:3) nicht messbar. Abbildung 1 zeigt das Aktivitätsverhalten der vier Katalysatoren bei verschiedener Vorbehandlung. An $CuO/ZrO_2$-3:7 wurde mit einem MeOH:CO-Selektivitätsverhältnis von 40:60 (molekulares Verhältnis) eine höhere MeOH-Selektivität erreicht als an CuO/ZnO-3:7 (20:80) (Abb. 1).

Hydrierung von $CO_2$ über aktivierten amorphen Metallegierungen

Die Hydrierung von $CO_2$ wurde an folgenden Ausgangslegierungen durchgeführt:
   A)  $Cu_{70}Zr_{30}$ amorph, $Cu_{70}Zr_{30}$ kristallin (Beispiel 1)
   B) $Ni_{64}Zr_{36}$ amorph (Beispiel 2)
   C) $Pd_{25}Zr_{75}$ amorph (Beispiel 3)
Die Metallegierungen wurden nach dem Schmelzspinnverfahren in Form von Bändern mit einer Dicke von 30 $\mu$m hergestellt und für die Versuchszwecke in kleine Stücke, ca. 1 bis 10 mal 10 bis 40 mm zerteilt und in dieser Form in den Mikroreaktor eingefüllt. Die Legierungen wurden ausschliesslich "in situ", d.h. im Reaktandenstrom aktiviert.

Beispiel 1 $Cu_{70}Zr_{30}$ (amorph und kristallin)

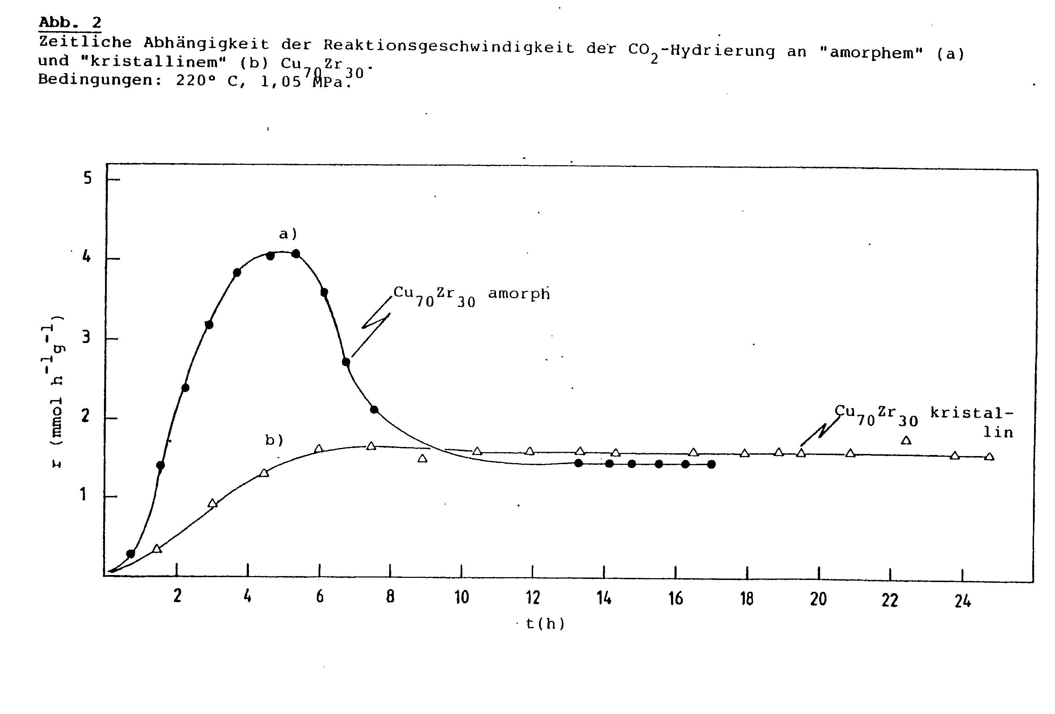

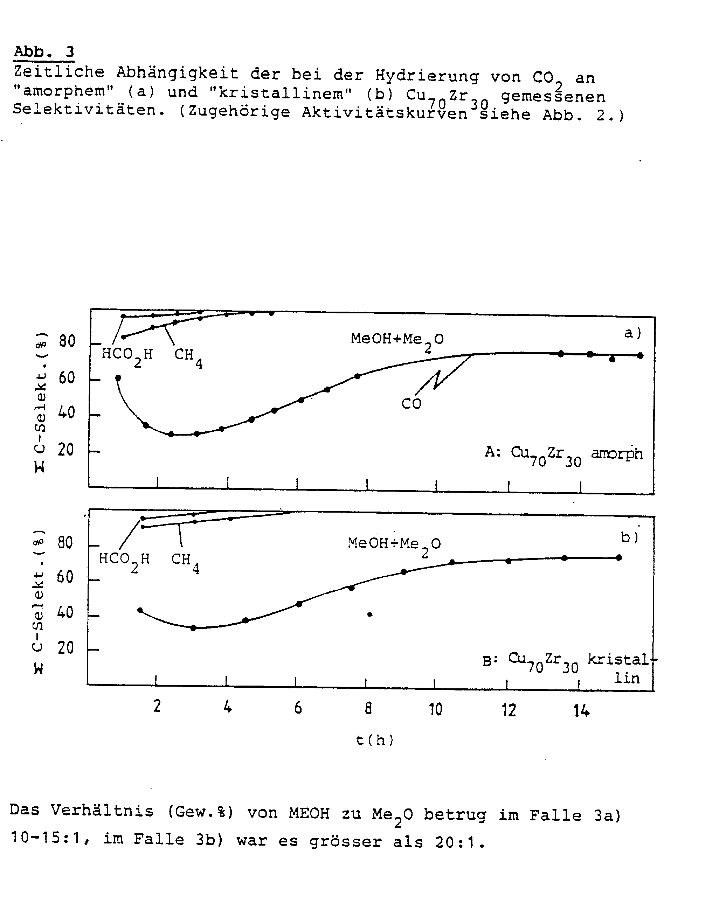

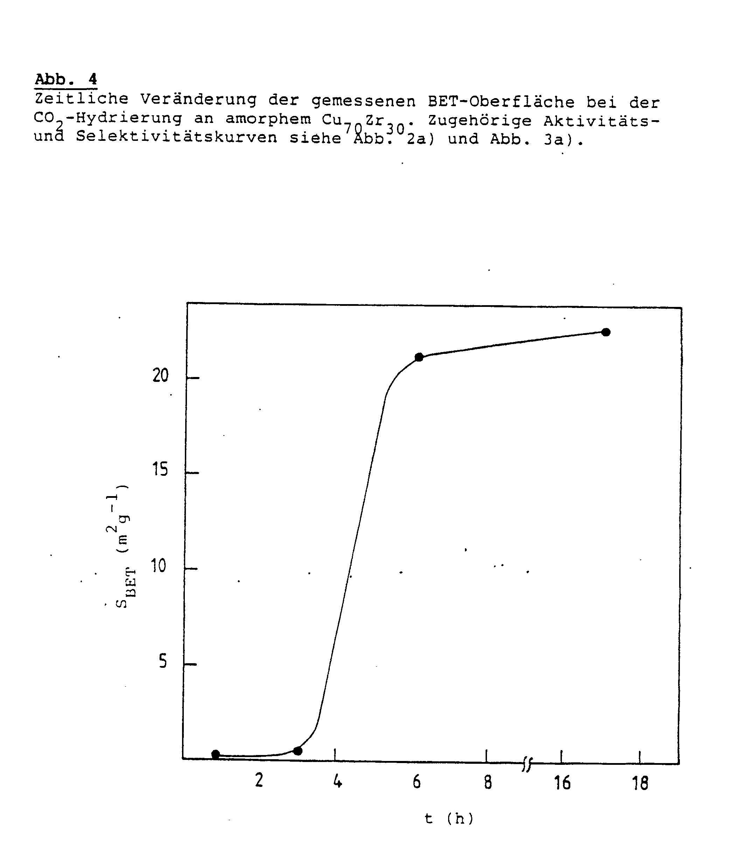

A) $Cu_{70}Zr_{30}$ (amorph) nach der Erfindung
Das Aktivität-Zeit-Diagramm der $CO_2$-Hydrierung an amorphem $Cu_{70}Zr_{30}$ (Abb. 2, Kurve a) zeigte nach einer anfänglichen Induktionsperiode ein ausgeprägtes Aktivitätsmaximum, welches nach 4,5 Stunden erreicht wurde; nach 10 Stunden wurde gleichbleibende Aktivität beobachtet. Eine Betrachtung der Selektivitäten (Abb. 3a) zeigte während der Anfangsphase ein dynamisches Verhalten. Die Selektivität von MeOH und $Me_2O$ erreichte nach 3 Stunden ein Maximum (grösser als 60 %), um nach ca. 10 Stunden auf einen konstanten Wert (grösser als 20 %) abzufallen. Neben der anfänglichen Bildung von Methan, sei besonders auf die Synthese von Ameisensäure hingewiesen.

Abbildung 4 zeigt die - während der "in situ"-Aktivierung der amorphen Probe - resultierende Vergrösserung der BET-Oberfläche. Röntgenographische Untersuchungen der resultierenden Katalysatoren zeigten, dass diese aus Kupferpartikeln bestanden, welche in eine oxidische Matrix von $ZrO_2$ eingebettet waren.

B) $Cu_{70}Zr_{30}$ (kristallin) Vergleichsbeispiel
Die Aktivität der $CO_2$-Hydrierung an kristallinem $Cu_{70}Zr_{30}$ durchlief kein ausgesprochenes Maximum (Abb. 2, Kurve b). Nach Durchlaufen der Induktionsperiode wurde nach 6 Stunden eine stationäre Aktivität beobachtet. Nach 10 Stunden wurde ein stationärer Zustand (d.h. gleichbleibende Aktivität und Selektivität) erreicht. Aehnlich wie bei amorphem $Cu_{70}Zr_{30}$ (Abb.3a) durchlief die MeOH- und $Me_2O$-Selektivität auch bei $Cu_{70}Zr_{30}$ kristallin nach 3 Stunden ein Maximum, und erreichte ca. 60 % (Abb. 3b). Auch bei der kristallinen Ausgangsprobe wurde während der Induktionsperiode eine signifikante Methan- und Ameisensäureproduktion beobachtet. Die Oberfläche vergrösserte sich von anfänglich 0,01 auf 25 m2/g nach 15 Stunden Reaktion. Das Röntgendiffraktogramm liess nach 15 Stunden $ZrO_2$ und kristallines Cu erkennen.

Ein Vergleich, der in der Abb. 2 dargestellten Aktivität-Zeit-Diagramme, mit denjenigen, welche für die konventionellen Vergleichskatalysatoren gemessen wurden (Abb. 1), zeigt, dass die Legierungskatalysatoren wesentlich aktiver sind. Beträgt das Aktivitätsverhältnis zum aktivsten CuO/ZnO-Fällungskatalysator 1:0,27, so ist es mit 1:0,03 erheblich grösser im Vergleich zum aktivsten CuO/ZrO_2-Fällungskatalysator (vgl. stationäre Aktivitäten in Abb. 1 und 2). Dies ist umso erstaunlicher, da CuO/ZrO_2-7:3 mit 57 m2/g nach der $CO_2$-Hydrierung eine mehr als doppelt so grosse Oberfläche hat wie $Cu_{70}Zr_{30}$(-amorph) nach dem $CO_2$ Hydrierversuch (22,7 m2/g).

Beispiel 2 $Ni_{64}Zr_{36}$ (amorph)

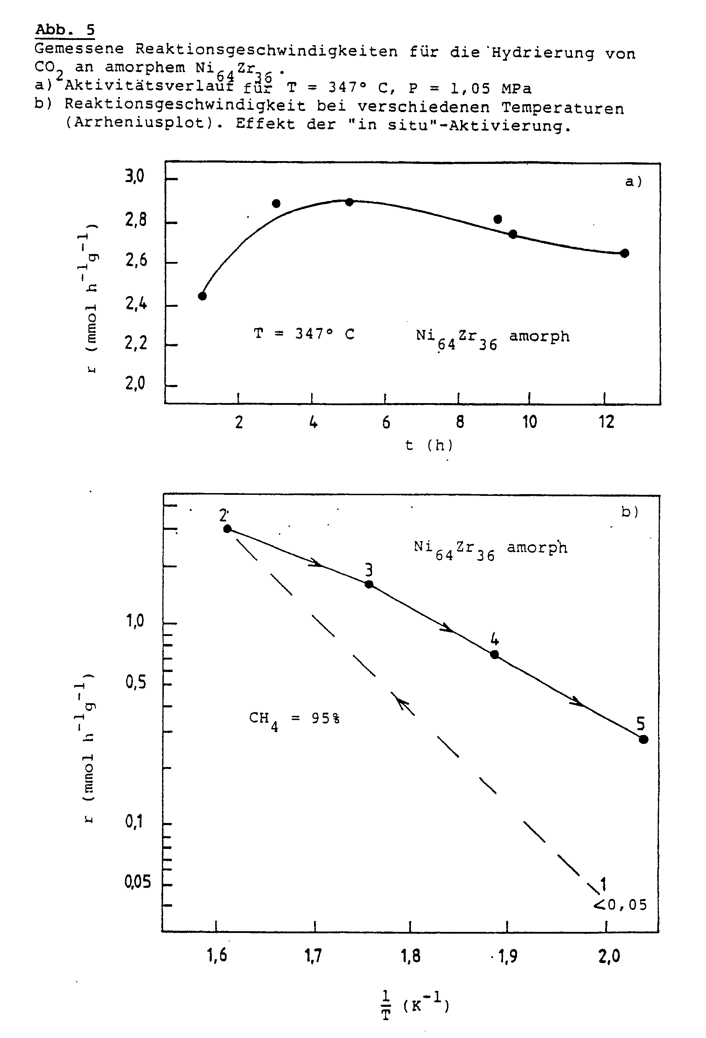

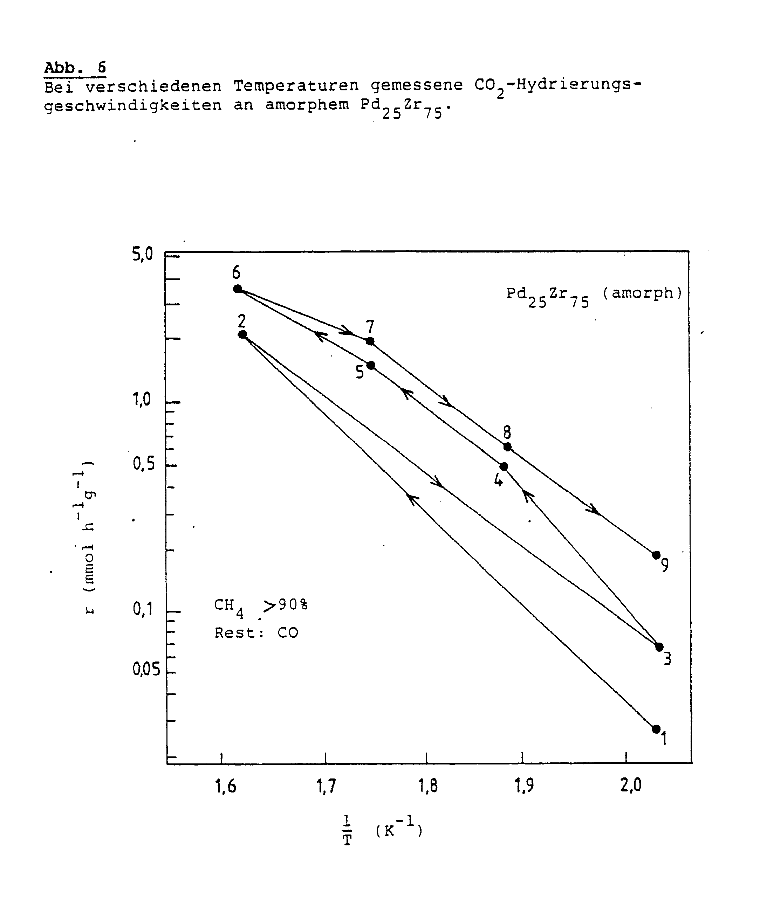

Bei der Hydrierung von $CO_2$ an $Ni_{64}Zr_{36}$ -(amorph) konnte bei 220°C und 0,1 MPa erst nur eine verschwindend geringe Aktivität gemessen werden. Bei 347°C stellte sich jedoch fast sofort eine quasistationäre Aktivität für die Methanbildung (Selektivität grösser als 95 %) ein (Abb. 5a). Bei nachfolgendem Senken der Temperatur auf 220°C liess sich auch bei dieser Temperatur eine signifikante Aktivität für die Methanbildung feststellen (Abb. 5b, Punkt 5). Nach diesem Prozedere zeigte das Röntgendiffraktogramm das Vorliegen von $ZrO_2$ und von kristallinem Ni. Die Oberfläche hatte sich von anfänglich 0,01 auf 22,1 m2/g vergrössert.

Beispiel 3 $Pd_{25}Zr_{75}$ (amorph)

Wie bei der Hydrierung von $CO_2$ an $Ni_{64}Zr_{36}$ konnte bei 220°C und 0,1 MPa erst nur eine geringe Methanbildung gemessen werden (Abb. 6, Punkt 1). Durch "in situ"-Aktivierung bei 345°C nahm die Aktivität stark zu, so dass bei nachfolgendem Senken der Temperatur auf 220°C (Punkt 3) eine wesentlich höhere Methanbildungsaktivität erreicht wurde als vor der Aktivierung. Zweimaliges Durchgehen dieses Aktivierungszykluses resultierte in einer Aktivität (Punkt 9), welche fast eine Grössenordnung höher war als die bei 220°C gemessene Anfangsaktivität (Punkt 1). Die Selektivität bezüglich Methanbildung war in jedem Versuch grösser als 90 %. Als Nebenprodukt trat CO auf.

**Patentansprüche**

1. Verfahren zur Hydrierung von Kohlendioxid in Gegenwart von Katalysatoren, dadurch gekennzeichnet, daß Kohlendioxid und Wasserstoff mit einem entweder bei 200 bis 350°C und $10^5$ bis $10^6$ Pa in einem Kohlendioxid und Wasserstoff in einem Verhältnis von Kohlendioxid zu Wasserstoff von 1:1 bis 1:4 Volumenteilen enthaltenden Gasstrom oder bei 200 bis 350°C im Wasserstoffstrom aktivierten Katalysator aus der Reihe der zirkonhaltigen Metallegierungen mikrokristalliner bis amorpher Struktur der Formel $Cu_{70}Zr_{30}$, $Ni_{64}Zr_{36}$ oder $Pd_{25}Zr_{75}$ in Berührung gebracht werden.

2. Verfahren nach Patentanspruch 1, dadurch gekennzeichnet, daß als Katalysator eine mikrokristalline bis amorphe $Cu_{70}Zr_{30}$ Metallegierung angewendet wird.

3. Verfahren nach Patentansprüchen 1 und 2, dadurch gekennzeichnet, daß als Katalysator eine amorphe $Cu_{70}Zr_{30}$ Metalllegierung angewendet wird.

4. Verfahren nach Patentanspruch 1, dadurch gekennzeichnet, daß als Katalysator eine amorphe $Ni_{64}Zr_{36}$ Metallegierungangewendet wird.

5. Verfahren nach Patentanspruch 1, dadurch gekennzeichnet, daß als Katalysator eine amorphe $Pd_{25}Zr_{75}$ Metallegierungangewendet wird.

6. Metallegierungs-Katalysator für die Hydrierung von Kohlendioxid mit mikrokristalliner bis amorpher Struktur und der Formel $Pd_{25}Zr_{75}$.

**Claims**

1. Process for hydrogenating carbon dioxide in the presence of catalysts, characterized in that carbon dioxide and hydrogen are contacted with a catalyst belonging to the series of zirconium containing metal alloys of microcrystalline to amorphous structure of the formula $Cu_{70}Zr_{30}$, $Ni_{64}Zr_{36}$ or $Pd_{25}Zr_{75}$ which catalyst has been activated either at 200 to 350°C and $10^5$ to $10^6$ Pa in a gas stream containing carbon dioxide and hydrogen in a ratio of 1:1 to 1:4 parts by volume or at 200 to 350°C in a hydrogen stream.

2. Process according to patent claim 1, characterized in that as catalyst a microcrystalline to

amorphous $Cu_{70}Zr_{30}$ metal alloy is used.

3. Process according to patent claims 1 and 2, characterized in that as catalyst an amorphous $Cu_{70}Zr_{30}$ metal alloy is used.

4. Process according to patent claim 1, characterized in that as catalyst an amorphous $Ni_{64}Zr_{36}$ metal alloy is used.

5. Process according to patent claim 1, characterized in that as catalyst an amorphous $Pd_{25}Zr_{75}$ metal alloy is used.

6. Metal alloy catalyst for the hydrogenation of carbon dioxide having a microcrystalline to amorphous structure and the formula $Pd_{25}Zr_{75}$.

## Revendications

1. Procédé d'hydrogénation du dioxyde de carbone en présence de catalyseurs, caractérisé en ce que du dioxyde de carbone et de l'hydrogène sont mis en contact avec un catalyseur de la série des alliages métalliques contenant du zirconium de structure microcristalline à amorphe de formule $Cu_{70}Zr_{30}$, $Ni_{64}Zr_{36}$ ou $Pd_{25}Zr_{75}$, activé soit à 200 jusqu'à 350°C et sous $10^5$ à $10^6$ Pa dans un courant gazeux contenant du dioxyde de carbone et de l'hydrogène dans un rapport du dioxyde de carbone à l'hydrogène de 1:1 à 1:4 parties en volume, soit à 200 jusqu'à 350°C dans le courant d'hydrogène.

2. procédé selon la revendication 1, caractérisé en ce que l'on utilise comme catalyseur un alliage métallique $Cu_{70}Zr_{30}$ microcristallin à amorphe.

3. Procédé selon les revendications 1 et 2, caractérisé en ce qu on utilise comme catalyseur un alliage métallique $Cu_{70}Zr_{30}$ amorphe.

4. Procédé selon la revendication 1, caractérisé en ce qu'on utilise comme catalyseur un alliage métallique $Ni_{64}Zr_{36}$ amorphe.

5. Procédé selon la revendication 1, caractérisé en ce que l'on utilise comme catalyseur un alliage métallique $Pd_{25}Zr_{75}$ amorphe.

6. Catalyseur de type alliage métallique pour l'hydrogénation du dioxyde de carbone, ayant une structure microcristalline à amorphe et de formule $Pd_{25}Zr_{75}$.

Abb. 1
Abhängigkeit der $CO_2$-Hydrierungsgeschwindigkeit von der Phasenzusammensetzung und den Aktivierungsbedingungen.
a) CuO/ZnO    Fällungskatalysator
b) CuO/ZrO$_2$   Fällungskatalysator
Experimentelle Bedingungen: T = 220° C, P = 1,05 MPa

**Abb. 2**

Zeitliche Abhängigkeit der Reaktionsgeschwindigkeit der $CO_2$-Hydrierung an "amorphem" (a) und "kristallinem" (b) $Cu_{70}Zr_{30}$.
Bedingungen: 220° C, 1,05 MPa.

**Abb. 3**

Zeitliche Abhängigkeit der bei der Hydrierung von $CO_2$ an "amorphem" (a) und "kristallinem" (b) $Cu_{70}Zr_{30}$ gemessenen Selektivitäten. (Zugehörige Aktivitätskurven siehe Abb. 2.)

Das Verhältnis (Gew.%) von MEOH zu $Me_2O$ betrug im Falle 3a) 10-15:1, im Falle 3b) war es grösser als 20:1.

**Abb. 4**

Zeitliche Veränderung der gemessenen BET-Oberfläche bei der $CO_2$-Hydrierung an amorphem $Cu_{70}Zr_{30}$. Zugehörige Aktivitäts- und Selektivitätskurven siehe Abb. 2a) und Abb. 3a).

**Abb. 5**
Gemessene Reaktionsgeschwindigkeiten für die Hydrierung von $CO_2$ an amorphem $Ni_{64}Zr_{36}$.
a) Aktivitätsverlauf für  T = 347° C, P = 1,05 MPa
b) Reaktionsgeschwindigkeit bei verschiedenen Temperaturen (Arrheniusplot). Effekt der "in situ"-Aktivierung.

**Abb. 6**
Bei verschiedenen Temperaturen gemessene $CO_2$-Hydrierungs-geschwindigkeiten an amorphem $Pd_{25}Zr_{75}$.